# EUROPEAN PATENT APPLICATION

(11) **EP 4 322 174 A1**
(43) Date of publication of application: **14.02.2024**
(21) Application number: 21936109.4
(22) Date of filing: 06.04.2021
(51) Int. Cl.: G16H 20/70, G16H 20/30, G16H 50/20, G16H 50/30, A61B 5/00

(54) **DIGITAL APPARATUS AND APPLICATION FOR TREATMENT OF MILD COGNITIVE IMPAIRMENT AND DEMENTIA**

(71) Applicant: S-Alpha Therapeutics, Inc., Seoul 06628 (KR)
(72) Inventor: CHOI, Seung Eun, Seoul 06628 (KR)
(74) Representative: Beck Greener LLP
(86) International application number: PCT/KR2021/004251
(87) International publication number: WO 2022/215764

(57) **Abstract**

A digital apparatus for treatment of mild cognitive impairment and dementia, according to an embodiment of the present invention, may comprise: a digital task generation unit which generates, on the basis of the pathogenesis and treatment hypothesis for mild cognitive impairment and dementia, a digital therapeutic module for treatment of the mild cognitive impairment and the dementia, generates a digital task embodied on the basis of the digital therapeutic module, and provides the digital task to a first user; and a result collection unit which collects results of the first user's performance on the digital task.

## Description

### Technical field

The present invention relates to a digital therapeutics module for the treatment and inhibition of the progress of amnestic mild cognitive impairment (MCI) and Alzheimer's disease (AD).

### Background art

Dementia refers to a clinical syndrome that impairs a person's ability to perform daily activities due to postnatal cognitive decline in memory, language, judgment, etc. Types of dementia are divided into degenerative dementia, which includes Alzheimer's disease, vascular dementia caused by stroke, etc., and others caused by various factors such as injuries, drugs, etc.

According to the "Year 2016 Nationwide Dementia Epidemiology Survey" (Report No. NIDR-1603-0015, 2017, June, Central Dementia Center, Ministry of Health and Welfare), the standardized dementia prevalence rate of male Korean elderly aged 65 or older was 8.18% and that of female was 10.46%, making a total of 9.50%. The data show that the prevalence rate of women was higher than that of men.

Further specifying the types of dementia from the standardized dementia prevalence rate among Korean elderly aged 65 or older (9.50%), AD accounted for 7.07% of the total, while vascular dementia accounted for 0.83%, and the others accounted for 1.60%. It is clear that AD accounts for an overwhelming proportion (approximately three-quarters) compared to other types of dementia.

Notably, there is an observable increase in the standardized dementia prevalence rate with the aging of the population. When comparing the standardized prevalence rate of Korean elderly aged 65 or older with that of Korean elderly aged 85 or older, the rate increases from 9.50% to 38.39%. In particular, the standardized prevalence rate of dementia among men aged 85 or older surges to 53.99%.

The dementia prevalence rate among the Korean elderly and the number of dementia patients estimated with the future population based on the age, sex, and region standardization of the 2015 population census is even more striking. The standardized dementia prevalence rate for those aged 65 or older in 2016 at 9.73% is expected to increase to 10.29% in 2020, 10.56% in 2030, and 16.09% in 2050. According to the estimated shift, the number of Korean dementia patients will be over one million by 2025 and over three million by 2050.

MCI (Korean Standard Disease and Sign Classification Code: F06.7), an intermediate state between normal aging and dementia, does not cause any difficulty in daily activities but results in a decline in memory and cognitive functions compared to people of the same age. The types of MCI are divided into amnestic MCI in which memory loss is the predominant symptom, and non-amnestic MCI, which causes a decline in non-memory cognitive functions. Because it is known that about half of the people with MCI will develop dementia, MCI, is receiving public attention in terms of preventing dementia.

According to the survey report, the standardized MCI prevalence rate for males Korean elderly aged 65 and older is 18.09%, and that for females is 25.28%, making a total of 22.25%. The data show that, as with the dementia, prevalence rate for females was higher than for males among those aged 65 and older.

Further specifying the types of MCI from the standardized MCI prevalence rate among Korean elderly aged 65 or older (22.25%), amnestic MCI accounted for 16.69% while non-amnestic MCI accounted for 5.56%. Amnestic MCI accounted for about three-quarters of the total. Amnestic MCI particularly requires more active care as it has a higher risk of developing into dementia, although it may not cause any difficulties in daily life.

Regarding the increase in prevalence rate, the yearly dementia care cost per patient and the government's budget allocation for dementia care should be noted. According to the Korean Dementia Observatory Report 2018 (2018, Dementia Care Center), the yearly dementia care cost per patient is 20.74 million KRW, and the government budget allocated for dementia care is 14.6 trillion KRW, accounting for 0.8% of the national GDP. The social costs associated with dementia are projected to surge dramatically escalating from a total investment of 17.9 trillion KRW in 2020 to an estimated 87.2 trillion KRW by 2050.

Globally, the world is facing the same challenges of increasing dementia prevalence rates and government budgets for dementia care, although the specifics may vary by region and population aging.

In the case of a vascular dementia patient, he or she may be cured by performing surgeries if the cause of dementia is due to stroke, brain tumor or normal pressure hydrocephalus (NPH). In the case of vascular dementia caused by brain infractions, the patient may prevent the disease or delay its progression by eliminating or managing elements such as hypertension, diabetes, smoking, hyperlipidemia, etc. However, it is hard to expect a positive therapeutic effect with the aforementioned methods from an AD, which is degenerative dementia, patient unlike those with vascular dementia.

Currently, the US FDA approves two types of drugs for AD treatment: cholinesterase inhibitors, such as Donepezil, Rivastigmine and Galantamine; and N-methyl-D-aspartate (NMDA) antagonist such as Memantine. Donepezil, which is a cholinesterase inhibitor, is generally prescribed to treat all stages of AD, and Rivastigmine and Galantamine to treat mild to moderate stage of AD. Memantine in combination with Donepezil is prescribed to treat moderate to severe stage of AD. However, the therapeutic effect of such drug treatment for AD is very limited. These drugs only serve to ease the symptoms rather than halting the progression of the disease or curing it. At the same time, the questions of the uselessness of drug treatment for dementia have been raised continuously as drug treatment entails serious and often fatal side effects and the fact that too many interactions between drugs may be dangerous has been pointed out. In fact, in France, insurance plans have suspended coverage for these four types of dementia treatment drugs since August 2018.

In addition, various cases where multinational pharmaceutical companies fail to develop or stop developing a cure for AD (e.g., Pfizer's Bapineuzumab; Eli Lilly's Solanezumab; Merck's Verubecestat, β-secretase (BACE) inhibitor; Boehringer Ingelheim's 'BI 409306'; AstraZeneca's Saracatinib; Biogen's Aducanumab; Novartis and Amgen's 'CNP520 (Umibecestat)'; and Roche's Crenezumab) demonstrate the difficulty in developing AD treatment. Hence, it is predicted that no innovative AD treatment will emerge in the near future.

As the limitations of drug treatment for dementia become clear, there are growing attempts to apply cognitive behavioral therapy (CBT), a non-pharmacological therapy used to ease mental health problems such as anxiety and depression, to treat dementia. In Korea, community-based cognitive stimulation training programs have been operated by Dementia Care Center, not by hospitals, because of insurance coverage issues. Essential cognitive rehabilitation programs include exercise therapy, reality awareness training, cognitive training therapy, reminiscence therapy, cognitive stimulation therapy, and music therapy. Other optional cognitive stimulation programs include occupational therapy, horticultural therapy, music therapy, art therapy, and exercise therapy.

To operate such programs, active interventionists such as clinical dietitians, exercise prescribers, physical therapists, etc., have to actively intervene and guide patients' behaviors. However, it is difficult for many patients to consistently access programs provided by high-quality interventionists due to issues such as interventionist skill levels, labor costs, turnover, and reimbursement economics.

As an alternative to overcome the CBT operation and financial difficulties, the National Health Service (NHS) in the United Kingdom provides a digital CBT service. The NHS recommends mobile applications for memory and mental health through App Library. For example, My House of Memories is an application that allows users to recall memories and store important memories in a memory tree, memory box, or timeline. Talk Around It Home is an application offering tests to name objects and games to improve word retrieval. MyCognition Home is an application offering brain training games based on the MyCQ test, which assesses planning ability, decision-making ability, memory, concentration, speed and accuracy.

Meanwhile, Dthera Sciences (OTCQB: DTHR) in the US is developing a digital treatment called DTHR-ALZ for neurodegenerative diseases, especially dementia, for the elderly, but it has not yet been approved by the FDA.

### Summary of Invention

### Technical task

It is an object of the present invention to provide a digital apparatus and an application for the treatment and inhibition of the progress of mild cognitive impairment (MCI) and Alzheimer's disease (AD) by deducing a mechanism of action (MOA) for amnestic MCI and AD based on the literature search and expert reviews of basic science reports and relevant clinical trial articles on the MOA of amnestic MCI and AD, and establishing therapeutic hypothesis and digital therapeutic hypothesis for the treatment and inhibition of the progress of amnestic MCI and AD based thereon.

Additionally, it is another object of the present invention to provide a digital apparatus and an application for a rational design of the application and for the treatment and inhibition of the progress of MCI and AD based thereon, so as to clinically verify the digital therapeutic hypothesis for the amnestic MCI and AD and implement the same as a digital therapeutic module.

Also, the present invention deduces the MOA, therapeutic hypothesis, and digital therapeutic hypothesis based on neuro-humoral factors of amnestic MCI and AD. It is yet another object of the present invention to provide a digital apparatus and an application capable of securing reliability of inhibiting the progress of amnestic MCI and AD and offering improved therapeutic effects through a patient's repetitive execution of digital instructions.

### Means for solving technical task

The digital apparatus for the treatment of MCI and dementia according to an embodiment of the present invention may include a digital instruction generation unit that generates, based on the MOA and therapeutic hypothesis of MCI and dementia, a digital therapeutics module for the treatment of the MCI and dementia, generates a specified digital instruction based on the digital therapeutics module, and provides the digital instruction to a first user, and a result collection unit which collects the first user's execution result of the digital instruction.

The digital application for treating MCI and dementia according to an embodiment of the present invention, as a digital application stored in a computer-readable medium, may instruct a computing apparatus to perform operations, comprising: generating a digital therapeutics module for treating the MCI and dementia based on the MOA and therapeutic hypothesis of MCI and dementia, generating a specified digital instruction based on the digital therapeutics module, providing the digital instruction to a first user, and collecting the first user's execution result of the digital instruction.

### Effect of invention

According to the digital apparatus and the application for the treatment and inhibition of the progress of amnestic MCI and AD of the present invention, the MOA, the therapeutic hypothesis, and the digital therapeutic hypothesis may be deduced considering neuro-humoral factors of the progression of amnestic MCI and AD, patients will be given digital instructions based on these findings, and the execution result thereof may be collected and analyzed in order to inhibit the progression of amnestic MCI and AD and offer improved therapeutic effects.

### Brief description of drawings

Fig. 1a is a diagram illustrating a MOA of AD proposed in the present invention, Fig. 1b a diagram illustrating a hypothesis for treatment and inhibition of the progress of amnestic MCI and AD proposed in the present invention, and Fig. 1c a diagram illustrating a digital therapeutic hypothesis for the treatment and inhibition of the progress of amnestic MCI and AD proposed in the present invention;
Fig. 2 is a block diagram illustrating the configuration of a digital apparatus for the treatment and inhibition of the progress of amnestic MCI and AD according to an embodiment of the present invention;
Fig. 3 is a diagram illustrating input and output loops of a digital application for the treatment and inhibition of the progress of amnestic MCI and AD according to an embodiment of the present invention;
Fig. 4 is a diagram illustrating a feedback loop according to a digital apparatus and an application for the treatment and inhibition of the progress of amnestic MCI and AD according to an embodiment of the present invention;
Fig. 5a is a diagram illustrating a module design implementing digital therapy in an apparatus and an application for the treatment and inhibition of the progress of amnestic MCI and AD according to an embodiment of the present invention, and Fig. 5b is a diagram illustrating a background factor supporting a digital apparatus and an application for the treatment and inhibition of the progress of amnestic MCI and AD according to an embodiment of the present invention;
Fig. 6 is a diagram illustrating a method for assigning a patient-customized digital prescription using a digital apparatus and an application for the treatment and inhibition of the progress of amnestic MCI and AD according to an embodiment of the present invention;
Figs. 7a to 7e illustrate examples of specific instructions and output data-collecting methods for each module according to an embodiment of the present invention;
Fig. 8 is a flow chart illustrating the operation of a digital application for the treatment and inhibition of the progress of amnestic MCI and AD according to an embodiment of the present invention;
Fig. 9 is a flow chart illustrating a method for generating digital instructions in a digital application for the treatment and inhibition of the progress of amnestic MCI and AD according to an embodiment of the present invention;
Fig. 10 is a flow chart illustrating repeated execution of operation according to a feedback control in a digital application for the treatment and inhibition of the progress of amnestic MCI and AD according to an embodiment of the present invention; and
Fig. 11 is a diagram illustrating a hardware configuration of a digital apparatus for the treatment and inhibition of the progress of amnestic MCI and AD according to an embodiment of the present invention.

### Best mode for carrying out the invention

Hereinafter, various embodiments of the present invention will be described in detail with reference to the accompanying drawings. In the description with reference to the accompanying drawings, the same or corresponding components are assigned the same reference numerals, and overlapping descriptions are omitted.

For the embodiments of the present invention disclosed herein, specified structural and functional descriptions are illustrated for the purpose of describing an embodiment of the present invention only, and various embodiments according to the present invention may be carried out in various forms and should not be construed as limited to the embodiments set forth herein.

The expressions "first," "second," etc., used in the embodiments may modify various components, regardless of order and/or importance, and do not limit the corresponding components. For example, a first component may be named a second component, or vice versa, without departing from the scope of the present invention.

The terms used herein are for the purpose of describing particular embodiments only and are not intended to limit the scope of other embodiments. As used herein, the singular forms are intended to include the plural forms, unless the context clearly indicates otherwise.

All terms used herein including technical or scientific terms may have the same meaning as commonly understood by those skilled in the art to which the present invention pertains. Terms such as those defined in a generally used dictionary may be interpreted to have the same or similar meanings as the contextual meanings in the relevant technology and should not be interpreted in an ideal or overly formal sense unless the context clearly indicates otherwise. In some cases, terms defined herein should not be interpreted to preclude the embodiments of the present invention.

Conventionally, the development of new drugs starts with confirming a medical demand in situ, proposing a MOA based on the expert reviews and meta-analysis on the disease, and deducing therapeutic hypothesis based on this. After a library of drugs whose therapeutic effects are expected is prepared based on the therapeutic hypothesis, a candidate substance is found through screening, and the corresponding substance is subjected to optimization and preclinical trials to verify its efficacy and safety from a preclinical stage, thereby deciding the candidate material as a final drug candidate. To mass-produce the corresponding drug candidate, a chemistry, manufacturing, and control (CMC) process is also established, and a clinical trial is carried out on the corresponding drug candidate to verify an MOA and therapeutic hypothesis of the drug candidate, thereby ensuring the clinical effectiveness and safety of the drug candidate.

From a patent perspective, drug targeting and signaling, which are upstream of new drug development, have many uncertainties. In many cases, because drug targeting and signaling require a methodology of compiling the results reported in the art, and interpreting the results, it may be difficult to guarantee the novelty of the invention. On the contrary, the invention of drugs capable of regulating the drug targeting and signaling to treat a disease requires the highest level of creativity, except in the field of some antibody or nucleic acid (DNA, RNA) therapeutics in spite of the development of research methodologies for research and development of numerous new drugs. As a result, the molecular structures of the drugs are the most critical factors that constitute product patents in the field of new drugs.

Unlike drugs that are strongly protected by product patents, a digital therapeutics module is basically implemented in software. Due to the nature of the digital therapeutics module, the rational design of the digital therapeutics module against the corresponding disease and its implementation thereof in software-based thereon may be considered to be a very creative process of invention that should be protected as a patent.

That is, the core of the digital therapeutics module as in the present invention depends on the rational design of the digital therapeutics module suitable for the treatment of the corresponding disease and the development of specified software capable of clinical verification based thereon. Hereinafter, a digital apparatus and an application for the treatment of MCI and AD of the present invention implemented in this light will be described in detail.

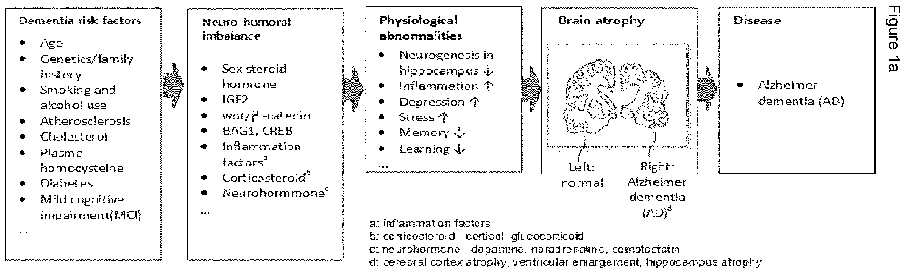

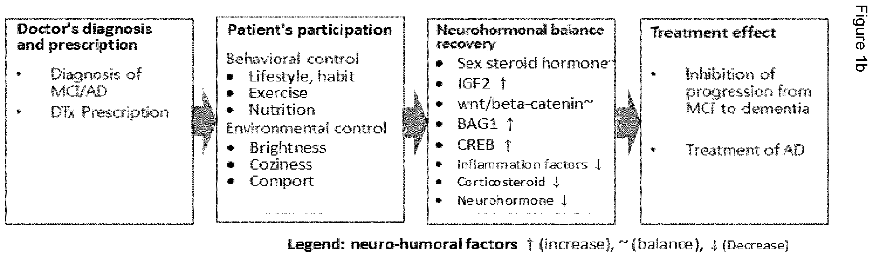

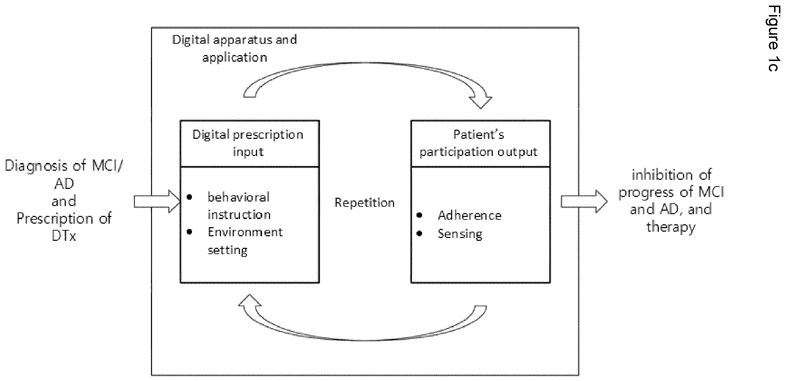

Fig. 1a is a diagram illustrating an MOA of AD proposed in the present invention, Fig. 1b is a diagram illustrating a hypothesis for treatment and inhibition of the progress of amnestic MCI and AD proposed in the present invention, and Fig. 1c is a diagram illustrating a digital therapeutic hypothesis for treatment and inhibition of the progress of MCI and AD proposed in the present invention.

The digital apparatus and application for the treatment and inhibition of the progress of MCI and AD of the present invention, described below, was implemented based on the MOA and therapeutic hypothesis deduced through the literature search and expert reviews of clinical trial articles on amnestic MCI and AD.

Generally speaking, disease therapy is carried out by analyzing a particular disease in terms of pathophysiological functions and dispositions in order to determine a starting point, a progression point, and an endpoint for the disease. Also, an indication of the disease is defined by demographic characteristics of the corresponding disease and statistical analysis of the disease. Furthermore, the patient's physiological factors, especially neuro-humoral factors, which correspond to the verified indications, are analyzed, and the patient's neuro-humoral factors are narrowly related to the disease to deduce the MOA.

Next, therapeutic hypothesis, in which the corresponding disease is treated by controlling actions and environments directly associated with the regulation of the corresponding neuro-humoral factors associated with the disease, is deduced. In order to implement this therapeutic hypothesis in a digital therapeutics module, a digital therapeutic hypothesis for achieving a therapeutic effect through repeated digital instructions and execution, which are associated with the "control of patient's action/environment regulation of neuro-humoral factors," is proposed. The digital therapeutic hypothesis of the present invention may be implemented as a digital apparatus and an application configured to present changes in the patient's actions (including behavioral, emotional, cognitive areas, etc.), improvement of the environment, and patient's participation in the form of specific instructions, and collect and analyze execution thereof.

The literature search for the clinical trials as described above may be performed through meta-analysis and data mining, and clinical specialist's feedback and in-depth reviews may be applied in each analysis step. Basically, the present invention may include extracting the MOA and therapeutic hypothesis for MCI and AD using the method described above, and regulating the neuro-humoral factors based thereon to provide a digital apparatus and an application as a digital therapeutics module for treatment and inhibition of the progress of MCI and AD.

However, the method of extracting the MOA and therapeutic hypothesis for MCI and AD is not limited to the methods described above, and MOAs and therapeutic hypotheses for diseases may be extracted using various methods.

Referring to Fig. 1a, various risk factors in the aging process such as age, heredity/family history, smoking and drinking, arteriosclerosis, cholesterol, serum homocysteine, diabetes, MCI and other factors may cause the imbalance of neuro-humoral factors in the aging process. For example, neuro-humoral factors, which may include abnormality in sex steroid hormone, decline in insulin-like growth factor-2 (IGF-2), abnormality in wnt/b-catenin, decline in BAG1, cAMP response element-binding protein (CREB), hypersecretion of corticosteroids such as inflammatory factors, cortisol and glucocorticoid, and hyposecretion of neuro-hormones such as dopamine, noradrenaline, and somatostatin, inhibit neurogenesis in the hippocampus in terms of physiological functions, may cause inflammation in brain tissue, and may also cause stress and depression. These physiological factors, over a long period of time, may induce plaque deposits, and neuronal apoptosis in the brain tissue, leading to brain atrophy with plaque deposits as a result, which is the anatomical characteristic of AD. As a result, a clinical syndrome commonly referred to as dementia occurs, which entails malfunctioning of brain, decline in cognitive functions such as memory, language, judgment, etc., and hence difficulties in performing activities of daily living.

Referring to Fig. 1b, the therapeutic hypothesis for MCI and AD according to the present invention relates to the treatment and inhibition of the progress of MCI and AD by restoring the balance of neuro-humoral factors through the patient's participation including the patient's behavioral control (e.g., lifestyle, habit, exercise, and nutrition) and environmental control (e.g., bright, familiar, relaxing execution environment setting).

Referring to Fig.1c, the digital therapeutic hypothesis for MCI and AD may be implemented by a digital apparatus and application which present the patient's participation including changes in the patient's action and improvement of the patient's environment in the form of specific instructions and collect/analyze execution thereof. When a digital therapeutics module of the present invention is used, the imbalance of neuro-humoral factors for amnestic MCI and AD patients may be corrected through the digital inputs (instructions) and outputs (execution) to achieve the treatment and inhibition of the progress of MCI and AD.

Meanwhile, Figs. 1a and 1b explain the MOA and therapeutic hypothesis for MCI and AD, but the present invention is not limited thereto. The methodology of the present invention may be applied to other types of MCI and dementia.

Also, although sex steroid hormone, IGF-2, wnt/b-catenin, BAG1, CREB, inflammatory factors, corticosteroids, and neurohormones are described as the neuro-humoral factors as shown in Figs. 1a and 1b, the description thereof is for illustrative purposes only, and the neuro-humoral factors are not limited thereto as for the MOA and therapeutic hypothesis of amnestic MCI and AD according to the present invention, and all neuro-humoral factors affecting MCI and dementia may be considered.

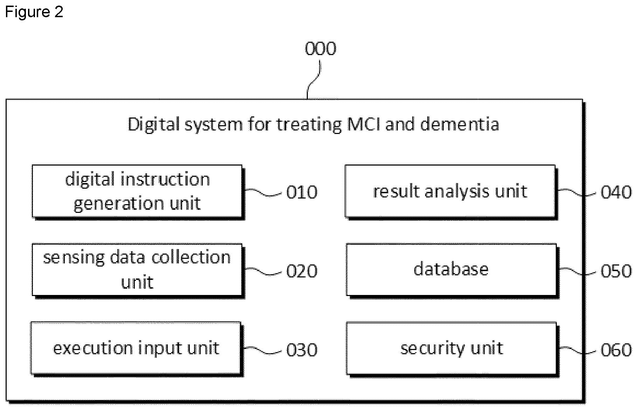

Fig. 2 is a block diagram illustrating the configuration of a digital apparatus for the treatment and inhibition of the progress of amnestic MCI and AD according to an embodiment of the present invention.

Referring to Fig. 2, a digital system 000 for treating MCI and AD according to an embodiment of the present invention may include a digital instruction generation unit 010, a sensing data collection unit 020, an execution input unit 030, a result analysis unit 040, database 050, and a security unit 060.

Based on the MOA, therapeutic hypothesis and digital therapeutic hypothesis for amnestic MCI and AD, a doctor (a second user) may prescribe a digital therapeutics module, which are implemented as a digital apparatus and an application for the treatment of MCI and AD to the corresponding patient. In this case, the digital instruction generation unit 010 may be configured to provide the digital therapeutics module prescription to a patient as a specified behavioral instruction that the patient may perform based on the interaction between the neuro-humoral factors for MCI and AD and the patient's behaviors/environments. For example, the neuro-humoral factors may include, but are not limited to, sex steroid horone, IGF-2, wnt/b-catenin, BAG1, CREB, inflammatory factors, corticosteroids, neuro-hormones, etc., and all types of neuro-humoral factors that may cause amnestic MCI and AD may be considered.

The digital instruction generation unit 010 may generate digital instructions based on the inputs from the doctor. In this case, the digital instruction generation unit 010 may generate digital instructions based on information collected by the doctor when diagnosing a patient (the first user). Also, the digital instruction generation unit 010 may generate digital instructions based on information received from the patient. For example, the information received from the patient may include the patient's basal factors, medical information, and digital therapeutics literacy. In this case, the basal factors may include the patient's activity, heart rate, sleep, diet (nutrition and calories), etc. The medical information may include the patient's electronic medical record (EMR), family history, genetic vulnerability, genetic susceptibility, etc. The digital therapeutics literacy may include the patient's accessibility to the digital therapy instructions and the apparatus, an acceptance posture, etc.

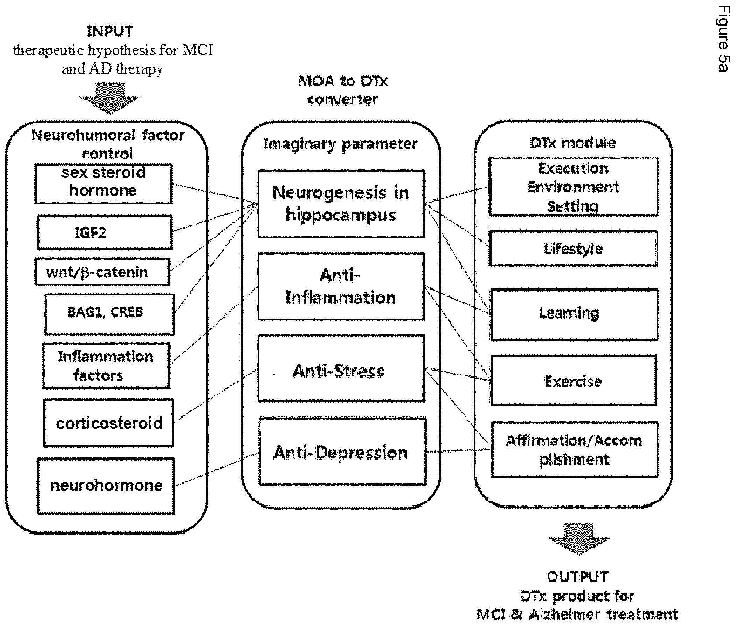

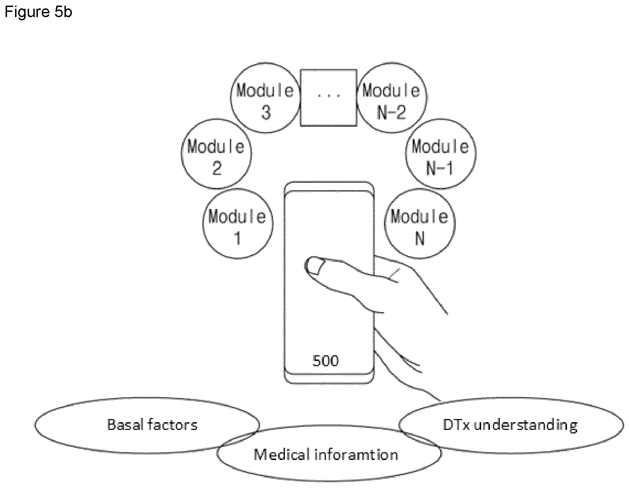

The digital instruction generation unit 010 may reflect the MOA and the therapeutic hypothesis for MCI and AD in order to utilize imaginary parameters and generate a digital module. In this case, the imaginary parameters may be deduced in terms of neurogenesis in the hippocampus, anti-inflammation, anti-stress and anti-depression, considering the patient's environment, behavior, emotion, and cognition. This will be described in detail in Fig. 5.

The digital instruction generation unit 010 may generate digital instructions specifically designed to allow a patient to have a therapeutic effect and provide the instructions to the patient. For example, the digital instruction generation unit 010 may generate specific digital instructions in each digital therapeutics module.

The sensing data collection unit 020 and the execution input unit 030 may collect the patient's execution result of the digital instructions provided through the digital instruction generation unit 010. Specifically, the sensing data collection unit 020 senses the patient's adherence to the digital instructions, and the execution input unit 030 allows a patient to directly input the execution result of the digital instructions, so as to output the patient's execution result of the digital instructions.

The result analysis unit 040 may collect the patient's behavioral adherence or participation in predetermined periods and report the same to the outside. Therefore, a doctor may continue to monitor an execution course of the digital instructions through the application even if a patient does not directly visit a hospital.

The database 050 may store the MOA and the therapeutic hypothesis of the MCI and AD, the digital instructions provided to the user, and data on the user's execution result. Fig. 2 illustrates that the database 050 may be included in the digital system 000 for treating MCI and AD, but the database 050 may be provided in an external server.

Meanwhile, a series of loops including inputting the digital instructions by the digital instruction generation unit 010, outputting the patient's execution result of the digital instructions by the sensor data collection unit 020/execution input unit 030, and evaluating by the result analysis unit 040 may be repeatedly executed several times. In this case, the digital instruction generation unit 010 may generate patient-customized digital instructions for the current cycle by reflecting the patient's digital instructions provided in the previous cycle, the output values, and the evaluation.

As described above, the digital apparatus for the treatment and inhibition of the progress of amnestic MCI and AD of the present invention may inhibit the progress of amnestic MCI and AD and provide improved therapeutic effects by deducing the MOA, therapeutic hypothesis, and the digital therapeutic hypothesis of MCI and AD based on the neuro-humoral factors of the progress of amnestic MCI and AD, providing digital instructions to the patient based thereon, and collecting and analyzing the execution result thereof.

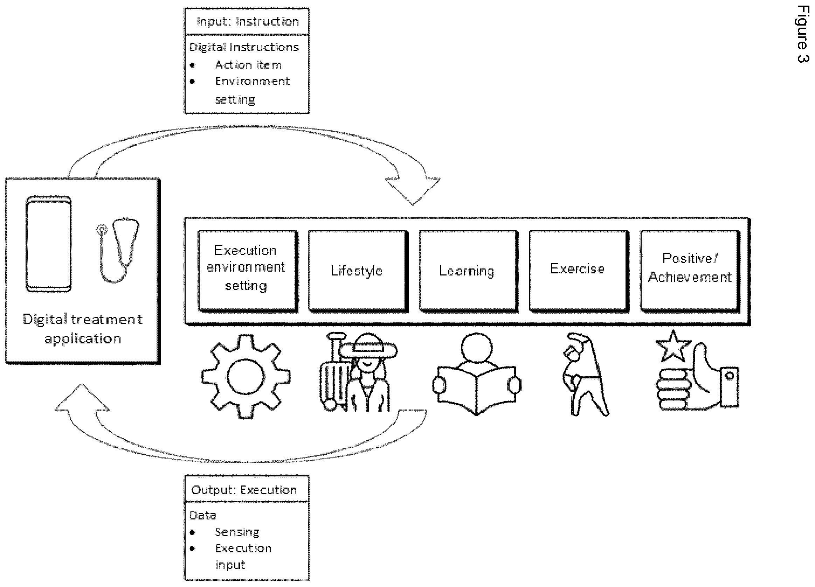

Fig. 3 is a diagram illustrating input and output loops of a digital application for the treatment and inhibition of the progress of amnestic MCI and AD according to an embodiment of the present invention.

Referring to Fig. 3, the digital application for treating MCI and AD according to an embodiment of the present invention may provide a corresponding digital prescription for a patient in the form of instructions. The result of the execution of the corresponding digital instructions may be reentered into the application as an output.

The digital instructions provided to the patient may include specific action instructions and control of the patient's environment. As illustrated in Fig. 3, the digital instructions may include execution environment setting, lifestyle, learning, exercise, and positive/achievement modules. However, the description thereof is for illustrative purposes only, and the digital instructions according to the present invention are not limited to these modules.

The patient's execution result of the digital instructions may comprise: 1) log-in/log-out information for instructions and execution, 2) adherence information sensed as passive data such as exercise, heart rate associated with stress, and change in oxygen saturation, and 3) directly input information of the patient's execution result.

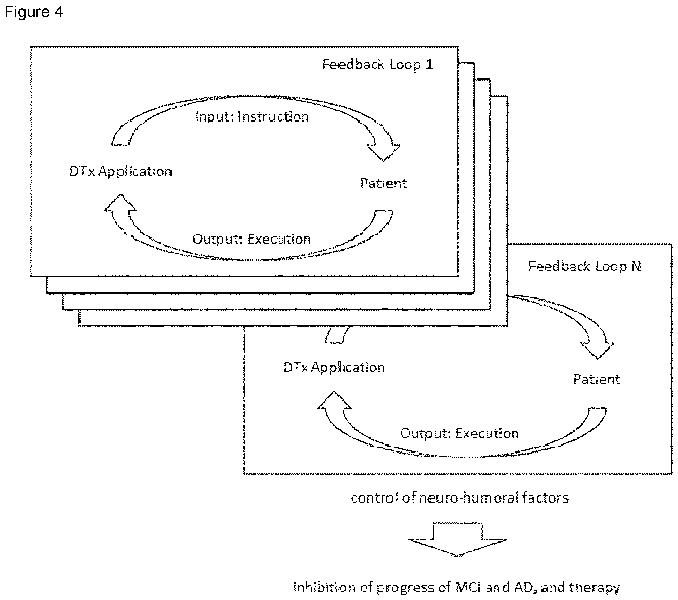

Fig. 4 is a diagram illustrating a feedback loop according to a digital apparatus and an application for the treatment and inhibition of the progress of amnestic MCI and AD according to an embodiment of the present invention.

Referring to Fig. 4, the treatment and inhibition of the progress of MCI and AD may be achieved by repeatedly executing the above-mentioned single feedback loop of Fig. 3 multiple times to regulate the neuro-humoral factors.

In the case of MCI and AD, due to the characteristics of these diseases, constant digital therapy and observation are required for treatment. Due to these characteristics, the effects of treatment and inhibition of the progress of MCI and AD may be achieved more effectively through gradual improvement of an instruction-execution cycle in the feedback loop, compared to the simple repetition of an instruction-execution cycle during the corresponding course of therapy.

For example, the digital instructions and execution result for the first cycle are given as input values and output values in a single loop, but new digital instructions may be generated by reflecting the input values and output values generated in this loop using a feedback process of the loop to adjust the input for the next loop when the feedback loop is executed N times. This feedback loop may be repeated to deduce patient-customized digital instructions while maximizing therapeutic effects.

As such, in the digital apparatus and the application for treating MCI and AD according to an embodiment of the present invention, the patient's digital instructions provided in the previous cycle (e.g., (N-1)th cycle) and the data on instruction execution results may be used to calculate the patient's digital instructions and execution results in the current cycle (e.g., N^{th} cycle). In other words, the digital instructions in the next loop may be generated based on the patient's digital instructions and instruction execution results calculated in the previous loop. In this case, various algorithms and statistic models may be used for the feedback process, if necessary.

As described above, in the digital apparatus and the application for the treatment and inhibition of the progress of MCI and AD according to an embodiment of the present invention, it is possible to optimize the patient-customized digital instructions suitable for the patient through the rapid feedback loop.

Fig. 5a is a diagram illustrating a module design implementing digital therapy in an apparatus and an application for the treatment and inhibition of the progress of amnestic MCI and AD according to an embodiment of the present invention, and Fig. 5b is a diagram illustrating a background factor supporting a digital apparatus and an application for the treatment and inhibition of the progress of amnestic MCI and AD according to an embodiment of the present invention.

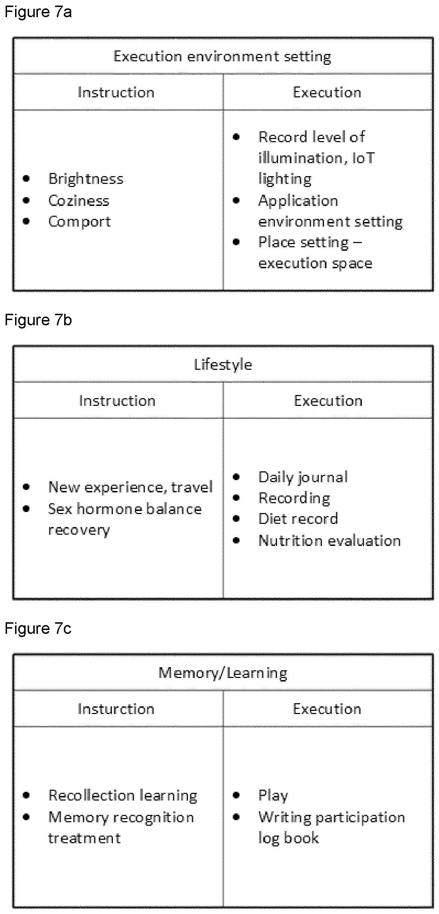

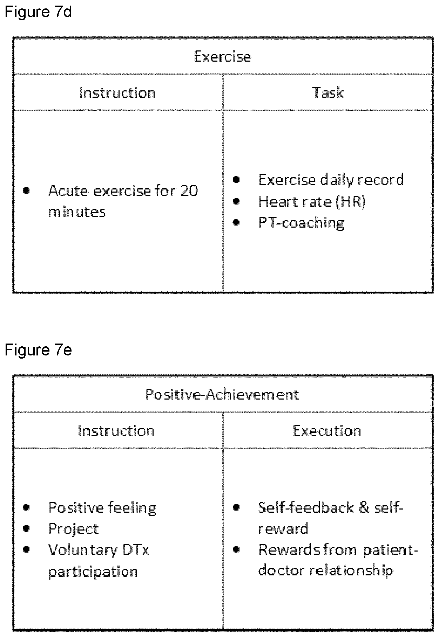

As illustrated in Fig. 5a, when the therapeutic hypothesis based on the MOA of MCI and AD is created, targeted neuro-humoral factors (e.g., sex steroid hormone, IGF-2, wnt/b-catenin, BAG1, CREB, inflammatory factors, corticosteroids, and neurohormones) may be deduced. Imaginary parameters may be used to allow specific instructions to correspond to the regulation of these neuro-humoral factors. The modules required for treating MCI and AD may be deduced using the "neuro-humoral factor-imaginary parameter-digital therapeutics module" interrelation. Each module will be described in the form of modular instructions more specifically in Fig. 7 which will be mentioned later. In this case, each module is a basic design unit for a digital therapeutics module implemented in the actual digital apparatus or application and is a collection of specific instructions.

Specifically, referring to Fig. 5a, the neuro-humoral factors deduced based on the MOA and the therapeutic hypothesis for MCI and AD may include sex steroid hormone, IGF-2, wnt/b-catenin, BAG1, CREB, inflammatory factors, corticosteroids, and neurohormones. In order to treat MCI and AD, the imbalance of the corresponding neuro-humoral factors, i.e., the MOA of MCI and AD, needs to be solved by balancing sex steroid hormone, increasing secretion of IGF-2, balancing wnt/b-catenin, increasing secretion of BAG1 and CREB, decreasing secretion of inflammatory factors and corticosteroids and increasing secretion of neurohormones.

The control of each neuro-humoral factor may correspond to a digital therapeutics module using imaginary parameters such as neurogenesis in the hippocampus, anti-inflammation, anti-stress, and anti-depression. Also, specific digital instruction may be generated for each module based on the converted module. In this case, the digital instructions may include the execution environment setting and modules (lifestyle, learning, exercise, and affirmation/achievement modules), which may be output through the monitoring. However, the description thereof is for illustrative purposes only and the module according to the present invention is not limited thereto.

Meanwhile, referring to Fig. 5b, the background factors may be considered together in the module design of the digital apparatus and the application for treating MCI and AD according to an embodiment of the present invention.

Here, the background factors are elements necessary for correcting the clinical trial results during the process of verifying the clinical efficacy of digital therapy for MCI and AD according to the present invention. Specifically, in the background factors illustrated in Fig. 5b, the basal factors may include activity, heart rate, sleep, and diet (nutrition and calories). The medical information may include EMR, family history, genetic vulnerability and susceptibility, etc., written when a patient visits a hospital. The digital therapeutics literacy may include the patient's accessibility to the digital therapy instructions and the apparatus, and an acceptance posture.

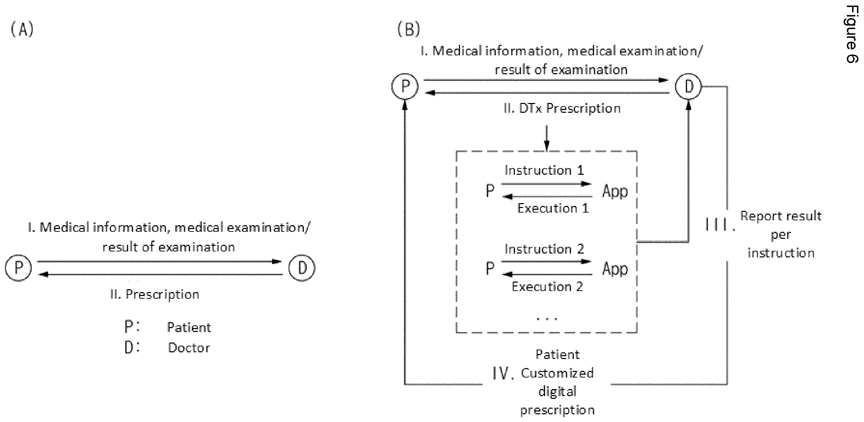

Fig. 6 is a diagram illustrating a method of assigning a patient-customized digital prescription using a digital apparatus and an application for the treatment and inhibition of the progress of amnestic MCI and AD according to an embodiment of the present invention.

Fig. 6(A) illustrates a prescription process through an ordinary medical examination between a patient and a doctor, and Fig. 6(B) illustrates a method of assigning a patient-customized digital prescription based on the analysis of a plurality of digital instructions and execution results thereof by the doctor.

As such, when using the digital apparatus and the application for treating MCI and AD according to an embodiment of the present invention, as illustrated in Fig. 6(B), the doctor may review the patient's instructions and execution results for a given period of time and adjust the types of modules for treating MCI and AD, and the instructions for each module in a patient-customized manner.

Fig. 7a illustrates the execution environment setting according to an embodiment of the present invention, and Figs. 7b-7e illustrate examples of specific instructions and output data-collecting methods for each module according to an embodiment of the present invention.

In the case of digital therapy for MCI and AD, since ongoing treatment is usually essential, it is important that participants feel interested in digital therapy and voluntarily take part in the therapy. In this context, the modules may be configured by adding game elements to each module. As described below, in the digital apparatus and the application for the treatment and inhibition of the progress of MCI and AD, each module is a basic design unit and a collection of specific instructions.

Fig. 7a illustrates examples of specific instructions and output data collection methods of the execution environment setting. In this case, the execution environment setting may be included as a configuration of the digital instruction generation unit 010 in Fig. 2. The other modules illustrated in Figs. 7b to 7e may be executed under a bright, familiar, and relaxing environment in the execution environment setting of Fig. 7a.

The brightness setting may include setting the brightness environment of the digital instruction execution environment utilizing an illuminance sensor or an loT lamp, and creating a living environment that is exposed to a brightness environment for at least 16 hours per day.

Familiarity setting may include setting the application environment (e.g., contents that might help the patient recall past memories, such as the patient's favorite songs, scenes and lines from the patient's favorite movies, family pictures, documentary photography, etc.) and the location setting configured to help the patient feel familiar while performing the instructions.

The relaxation setting, when executing the learning, and affirmation/achievement modules, may include an application environment setting (e.g., contents that might help relieve stress such as background wallpaper, music, tone, etc.) which provides a relaxing atmosphere for the patient to execute the instruction, and location setting which provides relaxation during the execution of instructions.

Figs. 7b to 7e illustrate examples of specific instructions and output data-collecting methods for each module according to an embodiment of the present invention. In the embodiments, modules may include lifestyle, learning, exercise, and affirmation/achievement modules.

Fig. 7b illustrates examples of specific instructions and output data-collecting methods for each module. In this case, the lifestyle module may be included as a configuration of the digital instruction generation unit 010 in Fig. 2.

The new experience and travel instructions may include a trip to a new place to experience new environments once every week or every other week. The new experience and travel (instructions) may include recording (execution) using journals or various digital media.

Balance of sex hormones instruction aims at recovering from the radical imbalance in sex steroid hormones due to aging and climacterium. Particularly, climacteric women who have undergone radical changes in sex hormones are advised to avoid hormone replacement therapy (HRT) or severe diets that involve radical fat loss. Regaining the balance of sex hormones (instructions) through a diet that reflects nutritional balance may include diet recording and nutrition assessment (execution).

Fig. 7c illustrates examples of specific instructions and output data-collecting methods for a learning module. In this case, the learning module may be included as a configuration of the digital instruction generation unit 010 in Fig. 2.

Specifically, the instructions of the learning module may include behavioral instructions that cause the patients to constantly repeat and recall familiar things in a relaxed atmosphere. In the case of the learning module, the first user's execution may be executed in the form of a game record and logbook for improving memory and learning. Additionally, a comfortable environment for associative learning may be provided through the background, tone, music settings, etc. of a digital therapeutics module. For example, the instructions from the learning module may direct the patient to listen to soothing music or to smell a pleasant scent.

Fig. 7d illustrates examples of specific instructions and output data-collecting methods for an exercise module. In this case, the physical exercise module may be included as a configuration of the digital instruction generation unit 010 in Fig. 2.

The exercise module may include a series of behavioral instructions involving 20 minutes of acute exercise on a regular basis (three times a week).

Specifically, the behavioral instructions of the exercise module may include a method of collecting the execution result with the sensing data collection unit 020 using biofeedback devices (e.g., measuring EEG, ECG, EMG, EDG, etc.) or general sensors (measuring activity, HR, etc.), or a method for the patient to directly input the execution result using the execution input unit 030. In this case, the exercise instructions of the present invention may be configured according to the age and physical condition of the patient, using the exercise therapy widely used by doctors and exercise therapists.

In the case of the exercise module, the first user may execute the behavioral instructions by writing exercise journals, checking heart rates, or getting a personal training (PT) coach, etc.

Fig. 7e illustrates examples of specific instructions and output data-collecting methods for affirmation/achievement modules. In this case, the affirmation/achievement modules may be configured as a configuration of the digital instruction generation unit 010.

Specifically, the affirmation/achievement modules may include instructions that stimulate the secretion of dopamine through the patient's execution of projects and the fulfillment of the completion. Here, a project completion instruction may be an instruction given to a patient that makes the patient feel a sense of accomplishment by completing the given project, wherein the project may be updated in accordance with the patient's participation period and may include games inducing voluntary participation. For example, the specific format of the game may take various forms such as learning, finding hidden objects or different pictures, quizzes, etc.

Particularly, that part implemented as a format of quiz from the affirmation/achievement module is further expected to enhance the patient's health information literacy and digital therapeutics literacy. Enhancement in health information literacy and digital therapeutics literacy is an essential element for a patient to continuously take part in the therapy and improve the performance status.

In the case of the affirmation/achievement module, the first user may execute the instruction in ways such as self-feedback, self-reward, or reward through the patient-doctor relationship, etc.

As mentioned above, the patient's long-term consistent or continuous participation is essential for digital therapy. Diligent participation in the instructions of the above-mentioned modules during this period may itself generate compliment (reward) instructions in the affirmation/achievement module so that the patient may feel a sense of accomplishment. In the compliment instruction, patients who actively participate in the therapy may be fed back with a sense of accomplishment through trust and reward between the patient-guardian and the patient-doctor.

Meanwhile, the progress of MCI and AD and aging are closely related. Particularly, in the old aging period, there is a wide gap in the standards set for new lifestyles, learning, exercise affirmation/achievement, depending on age, sex, personality, and preference. In order to overcome these gaps, it is preferable to propose customized digital instructions for each module in accordance with the individual characteristics of each patient. Particularly, the instructions that require intercommunication with the application may be developed by combining big data analysis and artificial intelligence analysis.

Each module and corresponding digital instructions illustrated in Figs. 7a to 7e are for illustrative purposes only, and the present invention is not limited thereto. The digital instructions provided to the patient may be set in various ways, as necessary.

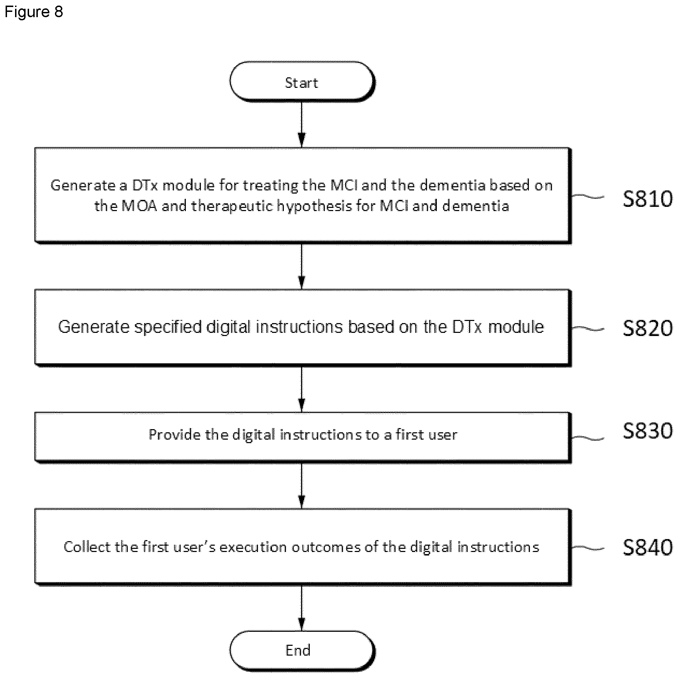

Fig. 8 is a flow chart illustrating the operation of a digital application for the treatment and inhibition of the progress of amnestic MCI and AD according to an embodiment of the present invention.

Referring to Fig. 8, the digital application for treating MCI and AD according to an embodiment of the present invention may first generate a digital therapeutics module for treating MCI and AD based on the MOA and therapeutic hypothesis of MCI and AD (S810). In this case, in step S810, the digital therapeutics module may be generated based on the neuro-humoral factors (e.g., sex steroid hormone, IGF-2, wnt/b-catenin, BAG1, CREB, inflammatory factors, corticosteroids, neurohormones, etc.) for MCI and AD.

Meanwhile, in step S810, a digital therapeutics module may be generated based on input from a doctor. In this case, the digital therapeutics module may be generated based on the information collected by the doctor when diagnosing a patient, and the prescription result written based thereon. Additionally, in the step S810, the digital therapeutics module may be generated based on the information (e.g., basal factors, medical information, digital therapeutics literacy, etc.) received from the patient.

Furthermore, in step S820, specified digital instructions may be generated based on a digital therapeutics module. In step S820, the digital therapeutics module may be generated by applying imaginary parameters about the patient's environment and behavioral aspects (e.g., neurogenesis in the hippocampus, anti-inflammation, anti-stress and anti-depression, etc.) to the MOA and therapeutic hypothesis for MCI and AD. This is explained in Fig. 5, and thus detailed description thereof will be omitted.

In this case, the digital instructions may be generated for at least one of the execution environment settings, lifestyle, learning, exercise, and affirmation/achievement modules. The execution environment setting and the specific digital instructions for each module are explained in Figs. 7a to 7e.

Next, the digital instructions may be provided to the patient (S830). In this case, the digital instructions may be provided in the form of digital instructions associated with behavior, emotion, cognition, etc., and in which the patient's instruction, adherence such as lifestyle and physical exercise may be monitored using a sensor, or in the form of digital instructions in which a patient directly enters the execution result.

After the patient executes the presented digital instructions, the patient's execution result of the digital instructions may be collected (S840). In step S840, as described above, the execution result of the digital instructions may be collected by monitoring the patient's adherence to the digital instructions or by allowing the patient to input the execution result of the digital instructions.

Meanwhile, the digital application for treating MCI and AD according to an embodiment of the present invention may repeatedly perform the steps of generating the digital instruction and collecting the patient's execution result of the digital instruction several times according to the feedback loop. In this case, the step of generating the digital instruction may include generating the patient's digital instruction for the current cycle based on the patient's digital instruction provided in the previous cycle and the execution result data of the patient's collected digital instruction.

As such, according to the digital method for the treatment and inhibition of the progress of amnestic MCI and AD of the present invention, the therapeutic hypothesis and the digital therapeutic hypothesis of MCI and AD may be deduced considering neuro-humoral factors of the progress of amnestic MCI and AD. Patients will be given digital instructions based on these findings, and their execution results will be collected and analyzed in order to effectively inhibit the progress of amnestic MCI and AD and offer improved therapeutic efficacy.

Although the digital apparatus and the application for treating MCI and AD according to an embodiment of the present invention have been described only with respect to the treatment of MCI and AD, the present invention is not limited thereto. Additionally, for the other diseases other than MCI and AD, digital therapy may be executed substantially in the same manner as described above.

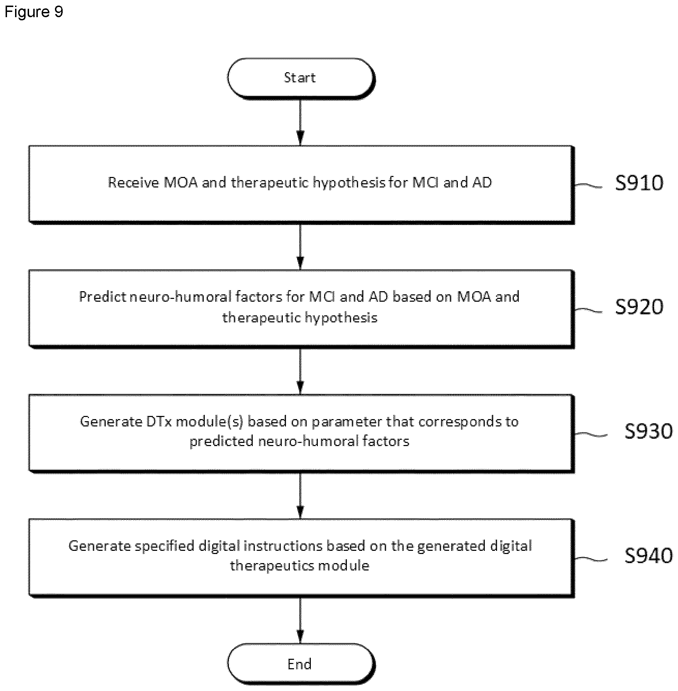

Fig. 9 is a flow chart illustrating a method of generating digital instructions in an application for the treatment and inhibition of the progress of amnestic MCI and AD according to an embodiment of the present invention.

Referring to Fig. 9, Fig. 9 illustrates the process of generating the module and the specified digital instructions for treating MCI and AD based on the MOA and the therapeutic hypothesis of MCI and AD (steps S810, S820 in Fig. 8), and the process of Fig. 5.

In step S910, the MOA and the therapeutic hypothesis for MCI and AD may be entered first. In this case, the MOA and the therapeutic hypothesis for MCI and AD may be deduced in advance through the literature search and expert reviews on the systematic related clinical trials on MCI and AD as described above.

Furthermore, neuro-humoral factors for MCI and AD may be predicted from the entered MOA and the therapeutic hypothesis (S920). In this case, the neuro-humoral factors for MCI and AD predicted by step S920 may be deduced in the form of sex steroid hormone, IGF-2, wnt/b-catenin, BAG1, CREB, inflammatory factors, corticosteroids, neurohormones, etc. These neuro-humoral factors have been explained in detail in Fig. 5, and thus detailed description thereof will be omitted.

In step S930, a digital therapeutics module may be generated based on the predicted neuro-humoral factors corresponding to the imaginary parameters. Here, the imaginary parameters may serve as converters that convert the neuro-humoral factors for MCI and AD into the digital therapeutics module, and this process is to establish the physiological interrelationship between the neuro-humoral factors and the patient's environmental and behavioral factors, as illustrated in Fig. 5.

Next, specific digital instructions may be generated based on the generated digital therapeutics module (S940). In this case, the specific digital instructions may be generated by the execution environment setting, lifestyle, learning, exercise, and affirmation/achievement modules explained in Figs. 7a to 7e.

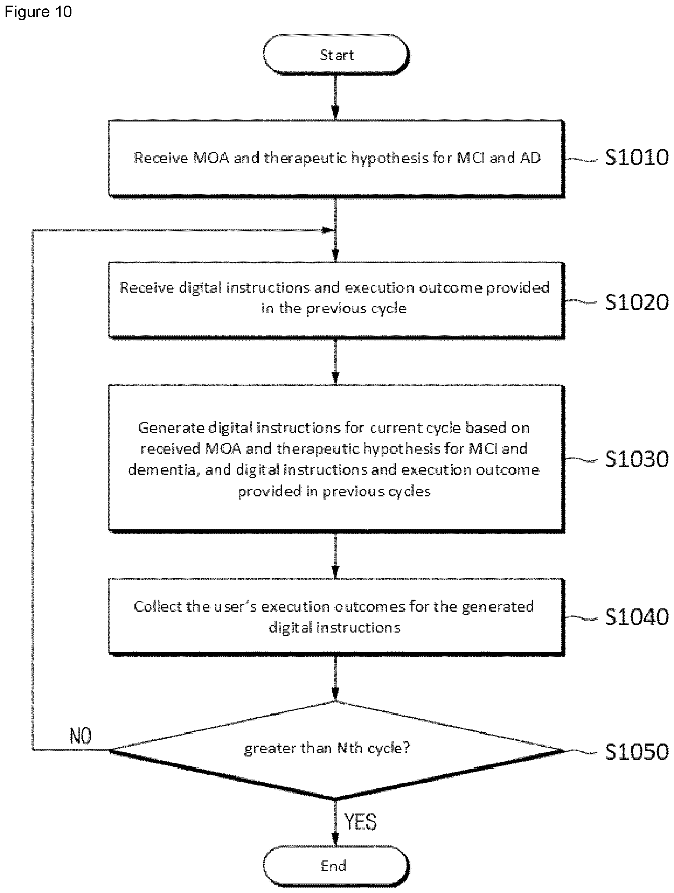

Fig. 10 is a flow chart illustrating the repeated execution of an operation according to feedback control in a digital application for the treatment and inhibition of the progress of amnestic MCI and AD according to an embodiment of the present invention.

Fig. 10 explains that the generation of digital instructions and the collection of the execution result by the application for treating MCI and AD are executed N times. In this case, first, the MOA and the therapeutic hypothesis for MCI and AD may be input (S1010). Additionally, the digital instructions and execution result data provided in the previous cycle may be received (S1020). If the current processing cycle is the first cycle, step S1020 may be omitted because there is no previous data.

Next, digital instructions for the current cycle may be generated based on the input MOA and therapeutic hypothesis, the digital instruction provided in the previous cycle, and the execution result data (S1030). Additionally, the user's execution result of the generated digital instructions may be collected (S1040).

In step S1050, it is determined whether the current cycle is greater than N^{th} cycle. If the current cycle is less than the N^{th} cycle (NO), this may return to step S1020, thereby repeating step S1020 to step S1040. On the other hand, if the current cycle is greater than the N^{th} cycle (YES), that is, if the generation of digital instructions and the collection of execution results are executed N times, the feedback operation may be terminated.

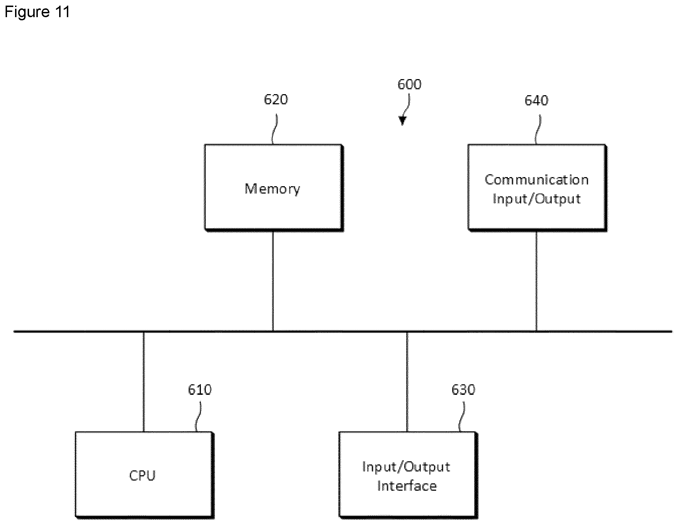

Fig. 11 is a diagram illustrating a hardware configuration of a digital apparatus for the treatment and inhibition of the progress of amnestic MCI and AD according to an embodiment of the present invention.

Referring to Fig. 11, hardware 600 of the digital apparatus for treating MCI and AD according to an embodiment of the present invention may include a CPU 610, a memory 620, an input/output I/F 630, and a communication I/F 640.

The CPU 610 may be a processor configured to execute a digital program for treating MCI and AD stored in the memory 620, process various data for the digital therapy for MCI and AD, and execute functions associated with the digital therapy for MCI and AD. That is, the CPU 610 may execute the digital program for treating MCI and AD stored in the memory 620 to execute functions of each constitution illustrated in Fig. 2.

The memory 620 may include a digital program for treating MCI and AD stored therein. Additionally, the memory 620 may include the data used for the digital therapy for MCI and AD contained in the above-mentioned database 050, for example, the patient's digital instructions, results of the execution of the instructions, the patient's medical information, etc.

A plurality of memories 620 may be provided, if necessary. The memory 620 may be a volatile memory or a non-volatile memory. If the memory 620 is a volatile memory, RAM, DRAM, SRAM, etc. may be used as the memory 620. If the memory 620 is a non-volatile memory, ROM, PROM, EAROM, EPROM, EEPROM, a flash memory, etc. may be used as the memory 620. Examples of the above-listed memories 620 are for illustrative purposes only, and the present invention is not limited thereto.

The input and output I/F 630 may provide an interface where input apparatuses (not illustrated) such as a keyboard, a mouse, a touch panel, etc., and output apparatuses such as a display (not illustrated) may be connected to the CPU 610 to transmit and receive data.

The communication I/F 640 is configured to transmit and receive various types of data to/from a server and may be one of various apparatuses capable of supporting wired or wireless communication. For example, various types of data concerning the above-mentioned digital behavior-based therapy may be received from a separately prepared external server via the communication I/F 640.

As such, the computer program according to an embodiment of the present invention may be recorded in memory 620 and processed by the CPU 610, for example, so as to be implemented as a module configured to execute each of the function blocks illustrated in Fig. 2.

Although all of the components configuring an embodiment of the present invention are described to be combined and operated together, the present invention is not necessarily limited to such an embodiment. That is, within the scope of the purpose of the present invention, the components may be selectively combined and operated.

In addition, the terms "including," "comprising," and "having" as used above indicate the presence of the corresponding components, but do not preclude the presence of other components unless specifically described to the contrary. All terms used herein, including commonly used technical and scientific terms, shall have the same meaning as generally understood by those skilled in the art, unless otherwise defined. Predefined, commonly used terms are interpreted to have the same meaning as the contextual meaning of the relevant technology and are not interpreted in an ideal or overly formal sense unless the context clearly indicates otherwise.

While the invention has been shown and described with reference to specific embodiments, it will be understood by those skilled in the art that various modifications and changes may be made therein without departing from the scope and spirit of the present invention. Accordingly, the embodiments disclosed in the present invention are not intended to limit but rather to illustrate the technical ideas of the present invention, and the embodiments do not limit the scope of the technical ideas of the present invention. The scope of protection of the present invention should be construed in accordance with the following claims, and all technical ideas within the same scope should be construed as being within the scope of the rights of the present invention.

## Claims

1. A digital apparatus for the treatment of mild cognitive impairment (MCI) and dementia, comprising:
a digital instruction generation unit which generates, based on a mechanism of action (MOA) and therapeutic hypothesis of MCI and/or dementia, a digital therapeutics module for the treatment of the MCI and dementia, generates a specified digital instruction based on the digital therapeutics module, and provides the digital instruction to a first user; and
a result collection unit which collects the first user's execution result of the digital instruction.

2. The digital apparatus of claim 1, wherein the digital instruction generation unit generates the digital therapeutics module based on neuro-humoral factors for the MCI and/or dementia.

3. The digital apparatus of claim 2, wherein the neuro-humoral factors include at least one of sex steroid hormone, insulin-like growth factor-2 (IGF-2), wnt/b-catenin, BAG1, cAMP response element-binding protein (CREB), inflammatory factors, corticosteroids, and neurohormones, wherein the corticosteroids include cortisol and glucocorticoid, and the neurohormones include dopamine, noradrenaline, and somatostatin.

4. The digital apparatus of claim 1, wherein the digital instruction generation unit generates the digital therapeutics module based on input from a second user.

5. The digital apparatus of claim 1, wherein the digital instruction generation unit generates the digital therapeutics module based on information received from the first user.

6. The digital apparatus of claim 5, wherein the information received from the first user includes at least one of basal factors, medical information, and digital therapeutics literacy of the first user, wherein the basal factors include the first user's activity, heart rate, sleep and diet (nutrition and calories), the medical information includes the first user's electronic medical record (EMR), family history, genetic vulnerability and genetic susceptibility, and the digital therapeutics literacy includes the first user's accessibility and acceptance posture to the digital therapeutics and the apparatus.

7. The digital apparatus of claim 1, wherein the digital instruction generation unit generates the digital therapeutics module by applying imaginary parameters to the MOA and therapeutic hypothesis of the MCI and dementia.

8. The digital apparatus of claim 7, wherein the imaginary parameters are deduced for at least one of the first user's neurogenesis in the hippocampus, anti-inflammation, anti-stress and anti-depression.

9. The digital apparatus of claim 1, wherein the digital instruction generating unit generates the digital therapeutics module according to at least one of execution environment settings, lifestyle, learning, exercise, and affirmation/achievement, and the corresponding digital instruction.

10. The digital apparatus of claim 9, wherein the digital instruction for the execution environment settings includes providing a familiar and relaxing instruction execution environment through a brightness environment setting, an application environment setting, and a location setting for the first user.

11. The digital apparatus of claim 9, wherein the digital instruction for the lifestyle includes at least one of providing new experience and travel to the first user and recovering balance in sex steroid hormones.

12. The digital apparatus of claim 9, wherein the digital instruction for learning includes at least one of associative learning and memory cognitive therapy for the first user.

13. The digital apparatus of claim 9, wherein the digital instruction for exercise includes the first user's acute exercise for a predetermined period of time.

14. The digital apparatus of claim 9, wherein the digital instruction for affirmation/ achievement includes at least one of providing an instruction for task achievement, assigning a project, and inducing voluntary participation in the project for the first user.

15. The digital apparatus of claim 1, wherein the result collection unit collects an execution result of the digital instruction by monitoring the first user's adherence to the digital instruction or allowing the first user to directly input the execution result of the digital instruction.

16. The digital apparatus of claim 1, wherein the generation of the digital instruction by the digital instruction generation unit, and the collection of the first user's execution result of the digital instruction by the result collection unit are repeatedly executed several times according to a feedback loop, and wherein the digital instruction generation unit generates the first user's digital instruction for a current cycle based on the first user's digital instruction provided in a previous cycle, and execution result data on the first user's digital instruction collected by the result collection unit.

17. A digital application for treating mild cognitive impairment (MCI) and dementia stored in a computer-readable medium, instructing a computing apparatus to execute operations, comprising:
generating a digital therapeutics module for treating MCI and dementia based on the mechanism of action (MOA) and therapeutic hypothesis of MCI and dementia;
generating a specified digital instruction based on the digital therapeutics module;
providing the digital instruction to a first user; and
collecting the result of the first user's execution of the digital instruction.

18. The digital application of claim 17, wherein the step of generating a digital therapeutics module generates the digital therapeutics module based on neuro-humoral factors for the MCI and dementia.

19. The digital application of claim 17, wherein the step of generating a digital therapeutics module generates the digital therapeutics module by applying imaginary parameters relating to at least one of the first user's neurogenesis in the hippocampus, anti-inflammation, anti-stress and anti-depression to the MOA and therapeutic hypothesis of the MCI and/or dementia.

20. The digital application of claim 17, wherein the step of generating a digital instruction generates the digital instruction for at least one of execution environment settings, lifestyle, learning, exercise, and affirmation/achievement.
